# EUROPEAN PATENT SPECIFICATION

(11) **EP 4 408 251 B1**
(45) Date of publication and mention of the grant of the patent: **09.04.2025**
(21) Application number: 22786805.6
(22) Date of filing: 22.09.2022
(51) Int. Cl.: A61B 1/005, A61B 1/00

(54) **AN ENDOSCOPE**
ENDOSKOP
ENDOSCOPE

(30) Priority: 28.09.2021 DK PA202170470
(43) Date of publication of application: 07.08.2024
(73) Proprietor: Ambu A/S, 2750 Ballerup (DK)
(72) Inventor: SILBERMANN, Victor, 2750 Ballerup (DK); MORTON, Alistair David, 2750 Ballerup (DK)
(74) Representative: AWA Denmark A/S
(86) International application number: PCT/EP2022/076418
(87) International publication number: WO 2023/052243

(56) References cited:
- JP-A- 2004 305 413
- US-A1- 2014 330 079
- US-A1- 2015 112 139

## Description

The present disclosure relates to an endoscope, in particular but not exclusively a single use insertion endoscope with a rotatable insertion cord. More specifically, the present disclosure relates to an endoscope comprising a proximal handle comprising a housing part and a distal insertion cord coupled to said proximal handle by means of a rotary coupling mechanism allowing rotation about a rotation axis.

In general, an endoscope comprises an operating handle at the proximal end and an insertion cord extending from the handle towards the distal end. The handle is adapted to be held by an operator and *inter alia* comprises externally protruding operating members connected to internal control gear allowing the operator to control the movement of a controllable bending section at the distal end of the insertion cord, while advancing the distal end of the insertion cord to a desired location e.g. within a body cavity of a person. By means of an attached monitoring device, such as a monitor with a display screen, the location to which the distal end has been advanced may be inspected using the endoscope.

The controllable bending section is normally an articulated section at the distal tip of the insertion cord that can be controlled by the operator from the handle, allowing the operator to advance the distal tip of the endoscope to a desired location by means of a series of actions involving inter alia bending the bending section in a desired direction, advancing the insertion cord and turning the insertion cord by turning the handle which is rigidly connected thereto. Having negotiated a tortuous path of bends and turns to a location of interest, the operator may end up in an awkward or ergonomically unfavourable body position.

This drawback may be overcome if the insertion cord is not rigidly connected to the handle, but may be turned with respect thereto. This allows the operator to turn the handle into a more convenient and ergonomically favourable position once the insertion cord is in place. It will also allow for an additional degree of freedom because the operator may choose to turn only the insertion cord rather than both the handle and the insertion cord when navigating during insertion.

In this respect US9307891 discloses an endoscope with a handle and an insertion cord that may be turned between a number of fixed latching positions with respect to the handle. There are no means to prevent turning the insertion cord too much with respect to the handle, just as statement that it should not be turned more than 180° in order not to break the inner workings i.e. the cables etc. running from the handle into the insertion cord. JP2004-305413 also discloses an endoscope with a handle and an insertion cord that may be turned with respect to the handle. The turning angle is limited by a stop so as not to twist and damage the fibre bundle running from the handle into the insertion cord when turning the insertion cord with respect to the handle. US 2015/112139 A1 discloses an endoscope according to the preamble of claim 1. US 2014/330079 A1 discloses an endoscope with an inner rotating cylinder positioned in the middle of the insertion section.

In recent years single use or disposable endoscopes that are used on one patient only before being thrown away have gained substantially in popularity as compared to traditional endoscopes that were reused after difficult and costly cleaning and sterilisation. Evidently, if an endoscope is single use it needs to be manufactured in a cost-efficient manner, meaning that the constituent parts should have low cost, and that the assembly thereof should be simple so as not incur unnecessary time consumption.

In this respect, the prior art couplings do not render themselves to cost-efficient assembly as it will be difficult to thread the flexible bundle of e.g. electrical supply wires and signals cables, pull wires, optical fibres, etc. forming part of a sub-assembly though these coupling mechanisms between the handle and the insertion cord.

Moreover, in use, the coupling should give the operator good tactile feedback. That is to say sufficient friction to avoid inadvertent turning of the insertion cord but not so much that it prevents smooth manipulation by the average operator.

Also, the cable bundle should be protected from damaging from e.g. twisting the insertion cord too much with respect to the handle.

Based on this it is the object of providing an endoscope having an insertion tube that may be turned with respect to the handle, and at the same time overcomes the above drawbacks.

According to the invention as defined in claim 1, this object is achieved by an endoscope comprising a proximal handle comprising a housing part and a distal insertion cord coupled to said proximal handle by means of a rotary coupling mechanism allowing rotation about a rotation axis, said coupling mechanism comprising a bushing having a through aperture defining an inner bushing wall surface adapted to receive a connection member and an outer bushing wall surface adapted to engage an inner handle surface in said handle, a connection member received in said through aperture and having a wall defining a through passage between a first end and a second end, wherein said second end is attached to an insertion cord sub-assembly, and said first end is rotatably coupled to a working channel part within said handle, wherein a lateral exit opening is provided in said wall between said first end and said second end comprises, and wherein a slit is provided in said wall from said second end to said exit opening.

This strongly facilitates the assembly of the endoscope because the entire insertion cord may be made or prefabricated as a separate insertion cord subassembly with all cables and wires extending from the proximal end. When attaching the proximal end of the insertion cord subassembly to the connection member the cables and wires need not be passed axially through any ports or apertures, but may just be passed laterally through the slit and emerge from the lateral exit opening. In other words, the cables and wires need not be threaded with the entire length with which they extend from the proximal end of the insertion cord subassembly through an eyelet. Rather, only a short length of the wires and cables need to be passed laterally through the slit which is a far easier procedure.

According to a preferred embodiment of the invention, the inner bushing wall surface comprises a rib protruding into said slit. Once the bushing has been passed over the insertion cord from the distal end this rib closes the slit in the connection member and keep the cables and wires centred between the distal end of the connection member and the lateral exit opening.

According to an embodiment of the invention, the coupling mechanism further comprises an annular gripping member having an external circumferential gripping surface and an inner gripping member surface, where the inner gripping member surface comprises a first inner gripping member surface part in engagement with said outer bushing wall surface and a second inner gripping member surface part rotatably engaging the exterior of said housing part. This allows a firm grip on the rotary part without having to grip the bushing wall itself which would normally be made of a low friction material less suitable for gripping.

According to an embodiment of the invention, the endoscope further comprises a locking clip securing said bushing against axial motion with respect to the housing part along said rotation axis.

According to an embodiment the locking clip is made from the same material as the bushing, so as to keep friction low.

According to an embodiment of the invention, the second inner gripping member surface part comprises at least one position reference member adapted to mutually engage a complementary position reference member on the exterior of said housing part. This allows for temporary rest in a neutral centre position and tactile feedback to the operator when moving from the neutral centre position.

According to a further preferred embodiment of the invention, the complementary position reference member is provided on a spring member provided integrally with the housing part, said spring member extending in a circumferential direction at the distal end of the housing part.

The disclosure will now be made in greater details based on non-limiting exemplary embodiments and with reference to the drawings, on which:
Fig. 1 shows a system comprising an endoscope according to the disclosure and a display device to which the endoscope may be connected,
Fig. 2 shows an exploded view of the endoscope of the system in Fig.1,
Fig. 2a shows an exploded view of the handle part of the endoscope of Fig 2 in greater detail and together with a connection cable,
Fig. 2b shows an exploded view of the insertion tube part of the endoscope of Fig. 2 in greater detail,
Fig. 3 shows the main handle housing part of Figs. 2 and 2a in even greater detail,
Fig. 4 shows the insertion tube part as an assembled insertion tube sub-assembly,
Figs. 5 and 6 show orthogonal views of the insertion tube sub-assembly and parts of the rotary coupling mechanism,
Fig. 7 shows an isometric view of a connection member,
Fig. 8 shows the connection of a connection member to a working channel tool insertion part in partial cross-section, and
Fig. 9 shows a cross-section of the working channel tool insertion part taken along the line IX-IX in Fig. 8,
Fig. 10 shows an isometric view from the distal end of a bushing
Fig. 11 is a longitudinal section in isometry from the opposite end of the bushing of Fig. 10,
Fig. 12 is a longitudinal section of the bushing taken along the line XII-XII in Fig. 11

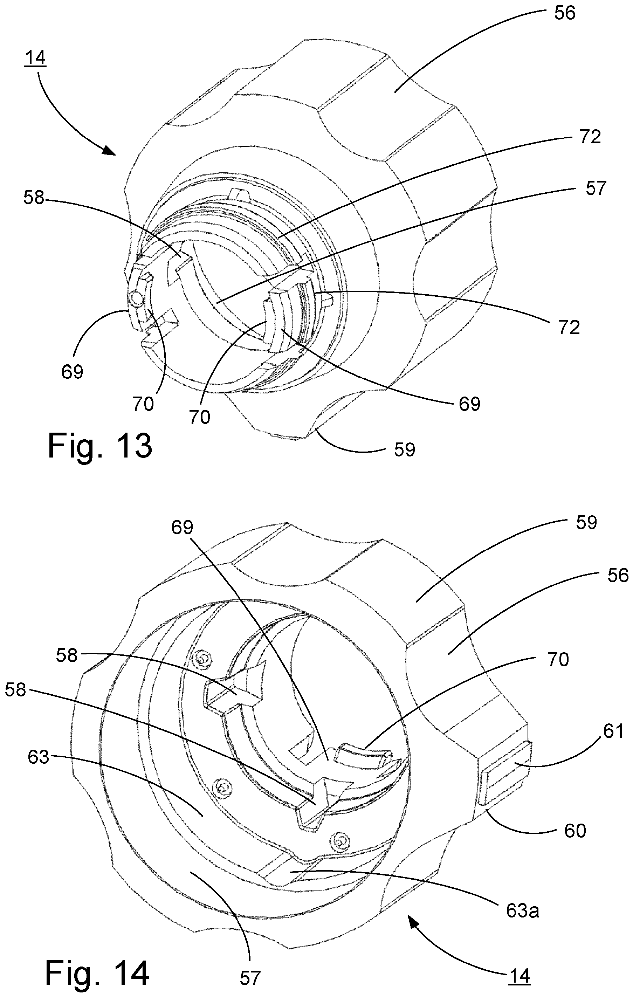

Fig. 13 shows an isometric view form the distal end of a gripping member,
Fig. 14 shows an isometric view form the proximal end of the gripping member of Fig. 13,
Figs. 15 and 16 show orthogonal cross-sectional views of the section of endoscope with the assembled rotary coupling mechanism,

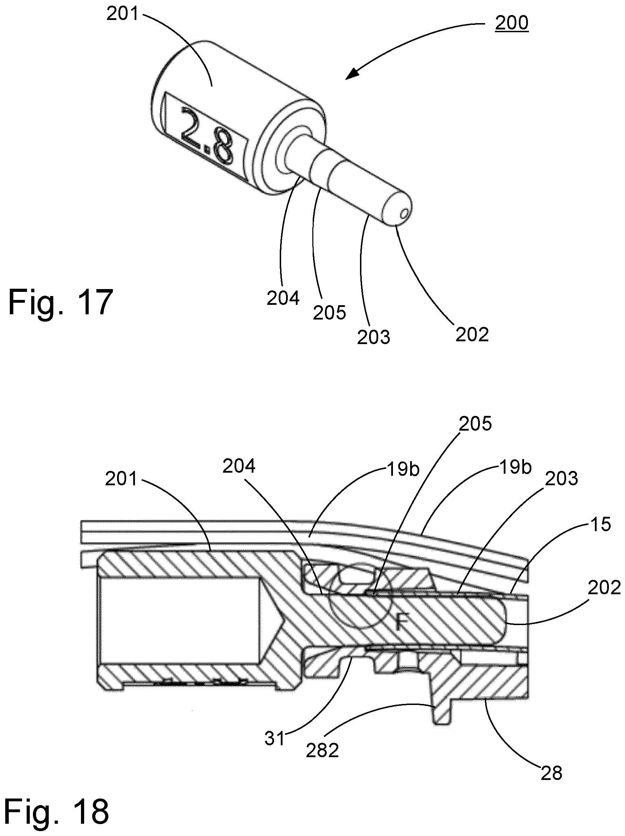

Fig. 17 shows a tool used in the mounting of the working channel tube, and
Fig. 18 a detail of the coupling mechanism in cross section with the tool in place.

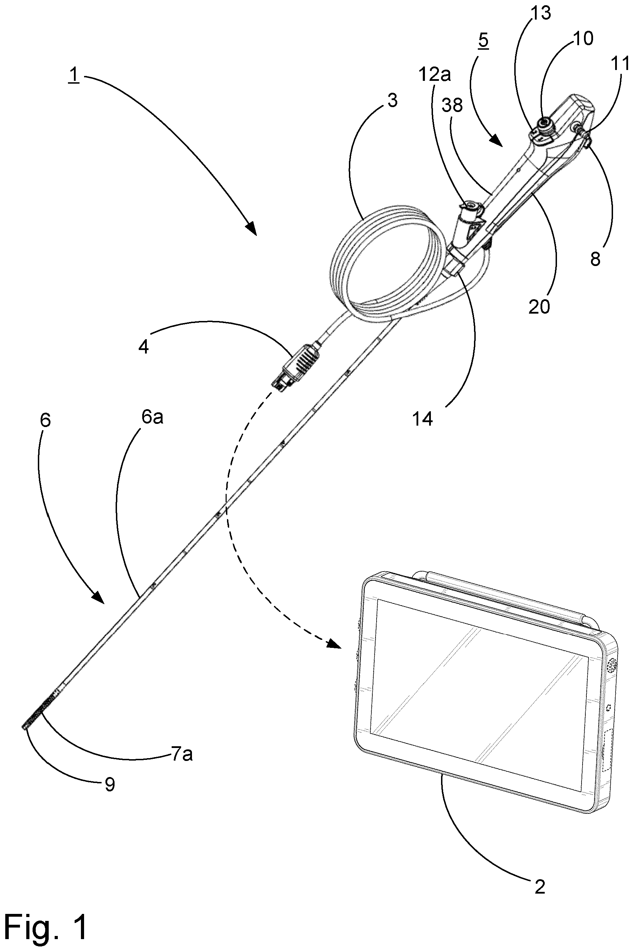

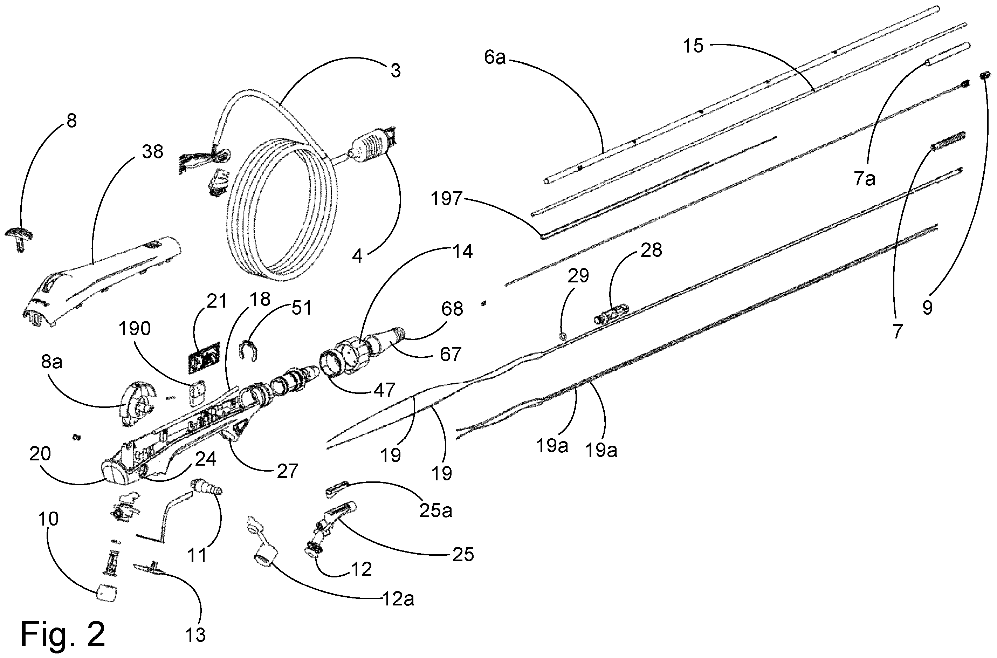

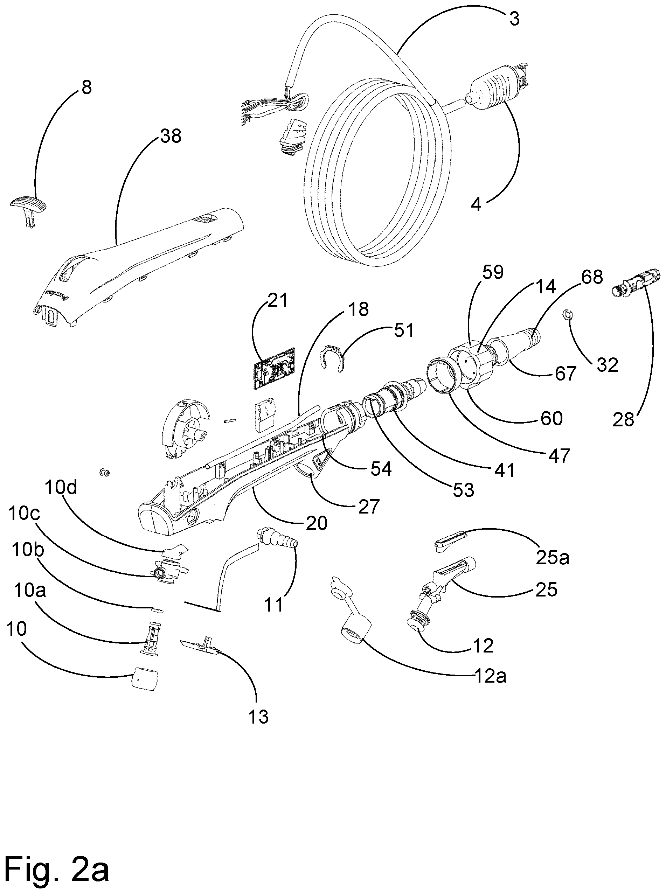

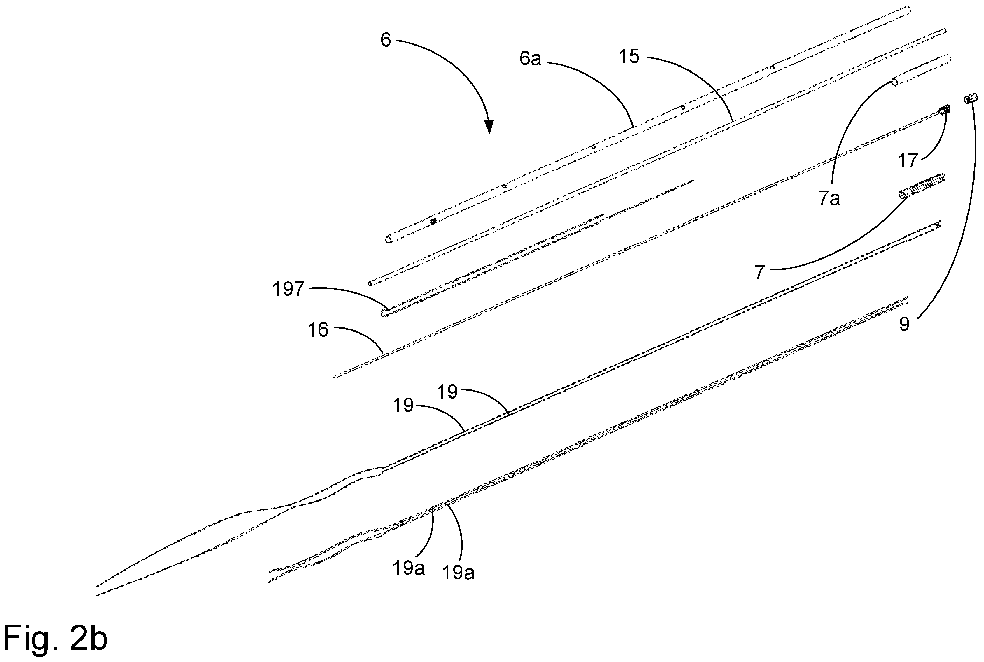

Turning first to Fig. 1 a system comprising an endoscope 1 and a display unit 2 is shown. The endoscope 1 may be connected to the monitor 2 by means of a cable 3 with a suitable connector 4. The endoscope 1 is preferably disposable i.e. intended to be thrown away after use on one single patient, whereas the display device 2 may be used multiple times with different reusable endoscopes. The endoscope is an insertion endoscope comprising a handle 5 at the proximal end, and an insertion cord 6 extending from the handle 5 towards the distal end of the endoscope 1. At the distal end of the insertion cord 6 a bending section which may be controlled by an operating member 8 is provided. As can be seen from Figs. 2 and 2b the bending section comprises a bending section body 7 beneath a flexible cover 7a. In turn at the distal end of the bending section 7 a tip housing 9 with the image capture device, lenses, illumination, the distal port of a working and/or suction cannel etc. of the endoscope is provided. At the distal end the working channel will normally serve dual functions as both working channels for tools and suction channel. The handle 5 normally also comprises a suction activation button 10 for activating the suction though the suction channel to an external vacuum source (not shown) via a suction connector 11. The handle may also have a tool insertion port 12 (cf. Fig 2) with a cap 12a for the insertion of an external tool through the handle and the working channel so as to emerge from the distal working channel port at the tip housing 9. Also, the handle 5 may be provided with buttons for activation electrical switches 13 controlling function of the image capture, such as taking of still images. In accordance with the present disclosure the insertion cord 6 may be rotated with respect to the handle 5 by manually gripping and turning a gripping surface 14 associated with a coupling mechanism which will be describe below.

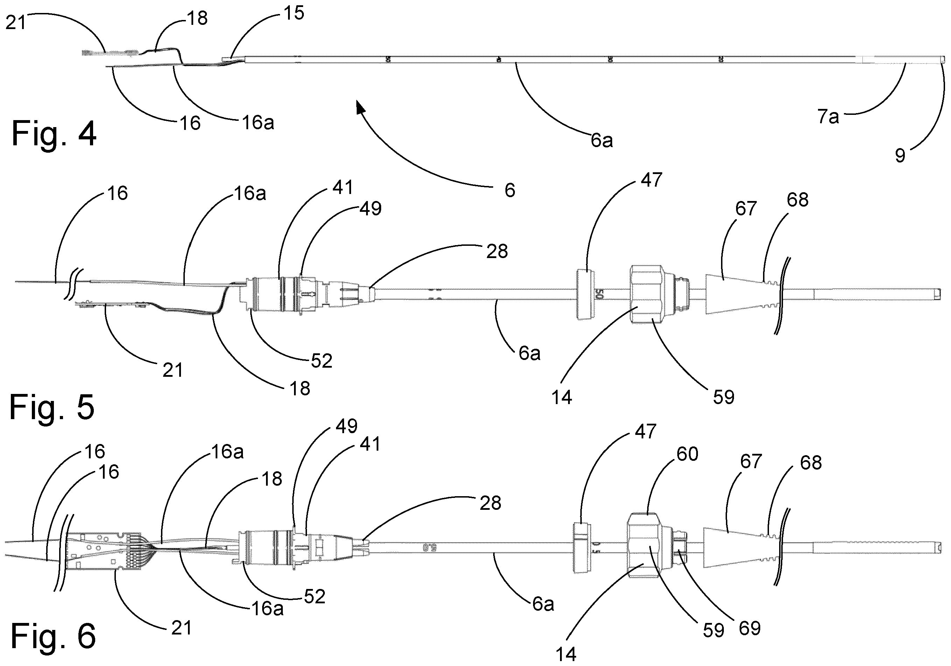

Turning now to Figs, 2, 2a and 2b the components of the exemplary embodiment of the endoscope 1 are shown in exploded view. In Fig. 2a the components of the handle 5, the coupling mechanism as well as the connection cable 3 are shown. In Fig 2b the components of the insertion cord sub-assembly are shown. These include the tip housing 9 attached to the bending section body 7 which is in turn connected to the main tube 6a forming the majority of the body of the insertion cord 6. Inside the main tube 6a a working channel tube 15 is provided. The working channel tube 15 passes through the bending section body 7 and is attached to the tip housing 9, more specifically so attached that it is in communication with a passage through the tip housing 9 to a distal working channel port. Electrical wiring 16 to the image capture device 17 also run along the inside of the main tube 6a, through the bending section body 7 and into the tip housing 9 where the image capture 17 device is accommodated. Likewise, the supply cables for the illumination components such as LEDs run along the inside of the main tube 6a, through the bending section body 7 and into the tip housing 9 where also the illumination components are accommodated. Similarly, the pull wires 19 for bending the bending section body 7 run along the inside of the main tube 6a and through the bending section body 7. To avoid ingress of water or the like the bending section body 7 is covered by a flexible cover 7a connected to the tip housing 9 and the main tube 6a in a sealed manner. A stabilizer 197 may also be accommodated in the main tube 6a. All these parts may be preassembled and the electrical wiring 16 and supply wires preferably also connected to a circuit board 21, as shown in Fig. 4 to provide a pre-assembled sub-assembly to be joined with the handle 5, in a subsequent assembly step when assembling the endoscope 1. As can be seen the electrical wiring 16, supply cables and pull wires 19 as well as the printed circuit board 21 extend from the sub-assembly. Accordingly, the handle 5 and the coupling mechanism according to the disclosure have been adapted for easy assembly despite these extending parts.

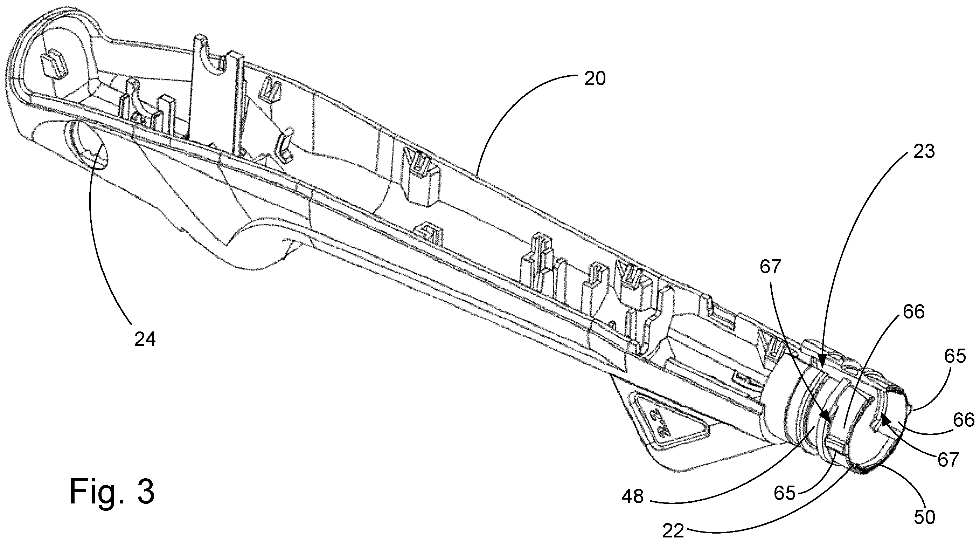

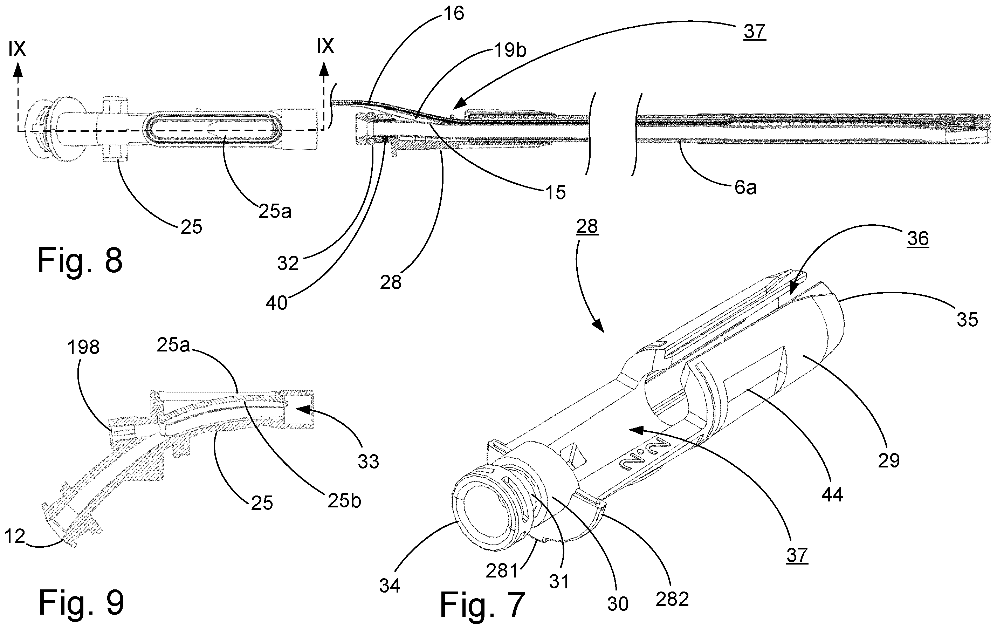

Turning now to Fig. 3 a handle housing part 20 is shown. The handle housing part 20 comprises a shell shape adapted for accommodating internal parts of the handle and is covered by a lid 38, cf. e.g. Fig. 2a, which is also somewhat shell-shaped. The handle housing part 20 serves as a base for supporting most of the internal parts accommodated in the handle 5, whereas the lid 38 essentially only serves as a closing cover. That is to say, the cover holds the operating member (8a), the anchoring block 190 for the guide tubes guide tubes 19a, a PCB 21 and the C-shaped clip 51 in position, but does preferably not carry or support any internal parts apart from that. As can be seen, the distal handle housing end 22 is C-shaped in circumference, with a longitudinal slit 23. As will be elaborated below, the distal handle housing end 22 is adapted for form part of a rotary coupling mechanism allowing the insertion cord 6 of the insertion cord sub-assembly (shown in Fig. 4) to be turned with respect to the handle 5. The handle housing part 20 also comprises an opening 24 adapted to receive the suction connector 11 for connecting the partially combined suction and working channel to an external vacuum source. The partially combined suction and working channel comprises a Y-junction body 25 comprising a tool entry port 12 located in a further opening 27 in the handle housing part 20. A further opening (not visible) may be provided in the handle housing part 20 to accommodate a suction activation valve assembly 10a, 10b, 10c, 10d, associated with the suction activation button 10 and connected to the suction connector 11. The suction activation valve assembly 10a, 10b, 10c, 10d is furthermore connected to one end of an intermediate suction tube 18, the other end of the intermediate suction tube 18 being connected to the Y-junction body 25, e.g. in a receptacle 198 formed in one branch of the Y-junction body 25, as indicated in Fig. 9. The Y-junction body 25 may be integrally formed but for manufacturing reason is preferably provides in two pieces, i.e. including a Y-junction lid 25a, with a guiding surface 25b facilitating the insertion of a tool from the tool port.

The working channel tube 15 located in the centre of the main tube 6a needs to be rotatable with respect to the distal end of the Y-junction body 25 which is fixed withing the handle 5. Pull wires 19, at least partially, need to be located off-centre to function. In any case, pull wires 19, electrical wiring 16 and supply cables for the LEDs cannot coincide with the working channel tube 15, and therefore must also be accommodated off-centre. Electrical wiring 16, supply cables and pull wires 19 may instead be made with overlength and slack so as to be able so as to allow them to twist and wrap. Accordingly, the pull wires 19 are preferably in guide tubes 19a to provide Bowden cables. To accommodate for all these requirements, the present disclosure utilises a connection member 28.

Fig. 7 shows the connection member 28 with a wall 29. At the proximal end the connection member 28 comprises a section 30 where the wall is circumferentially closed. In this section 30, an outer circumferential recess 31 adapted to accommodate sealing member 32, preferably in the form of an O-ring of an elastomer material, is provided. The outer diameter of the uncompressed sealing member 32 is slightly larger than the inner diameter of a cylindrical bore 33 provided in the distal end of the Y-junction body 25, so as to provide a seal allowing the connection member 28 to rotate with respect to the Y-junction body. The remainder of the wall 29 towards the distal end of the connection member 28 is not circumferentially closed. The wall 29 defining a through, but not circumferentially closed, passage between the proximal first end 34 and a distal second end 35. Towards the distal second end 35 the connection member 35 comprises a slit 36 in the wall 29. Between the slit 36 and the circumferentially closed section 30 of the wall 29 a larger opening 37 covering mostly half of the circumference is provided. As can be seen from the partial cross section of Fig. 8, this larger opening serves as an exit opening for the electrical wiring 16, supply cables and pull wires 19 covered with the guide tubes 19a.

During assembly of the endoscope the main tube 6a is inserted into the passage of the connection member 28 to the position shown in Fig. 8 where it is secured, preferably my means of an adhesive. During this insertion, the electrical wiring 16, supply cables and pull wires 19 extending from the proximal end of the main tube 6 of the insertion cord assembly may easily be passed through the slit 36 so as to emerge laterally from the exit opening 37, so that they will in the fully assembled endoscope pass around the outside of the Y-junction body inside the handle 5. This can be done even if the electrical wiring 16 and supply cables have been joined to the printed circuit board 21 in the pre-fabrication of the insertion cord assembly, because the circuit board need not pass through the connection member 28, and the electrical wiring 16 and supply cables need only pass the slit 36. The proximal end of the working channel tube 18 is adhered to the inside of the section 30 where the wall is circumferential, so as to provide a sealed connection. For this, a glue hole 40 for introducing the adhesive may preferably be provided in the otherwise circumferential wall section 30.

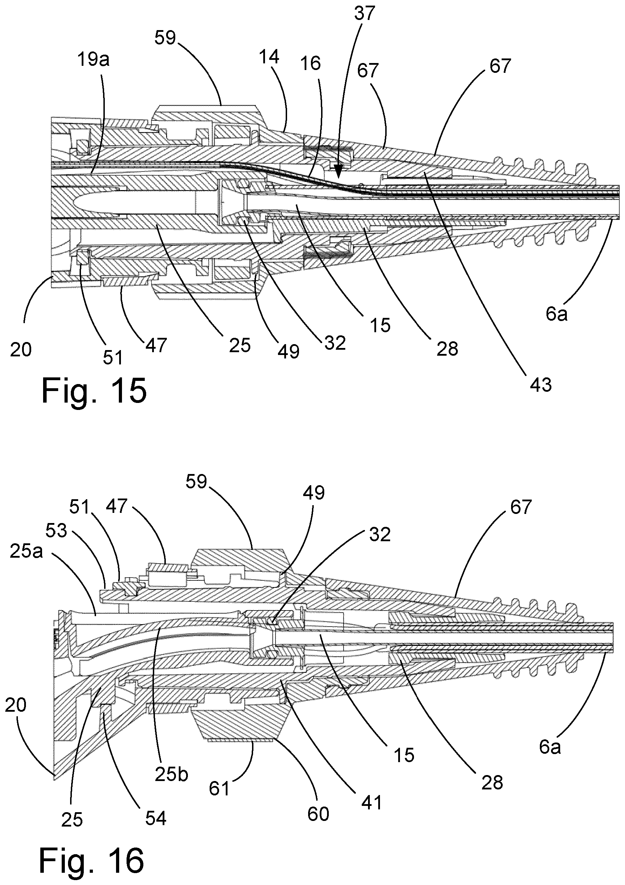

In order to ensure a smooth transition from the connection member 28 to the working cannel tube 15 the working channel tube 15 is preferably flared out at the proximal end. The smooth transition without edges is desirable to prevent procedure tools or optical fibres inserted through the Y-junction body 25 into the working channel 15 to be caught and disturbing the insertion process. For this a tool 200 as shown in Figs. 15 and 16. The tool has a gripping part 201 for digitally gripping it with thumb, index finger and possibly middle finger when inserting it into the connection member 28 and the working cannel tube 15 as shown in Fig. 16. The gripping portion 201 is preferably knurled or in other way prepared for good grip. The tool furthermore comprises an insertion section with a rounded distal insertion end 202, a first cylindrical part 203 of a first diameter and a second cylindrical part 204 of second diameter. The first diameter is larger than the second diameter. A tapering section 205, e.g. frusto-conical is provided between the second cylindrical part 204 and the first cylindrical part 203.

As can be seen from Fig. 16 the frusto-conical section of the tool expands the working channel tube 15 and provides a flare in the transition area marked (with the circle F) which is maintained once the glue 40 has set. It also helps aligning the working channel tube with the bore of the connection member 28.

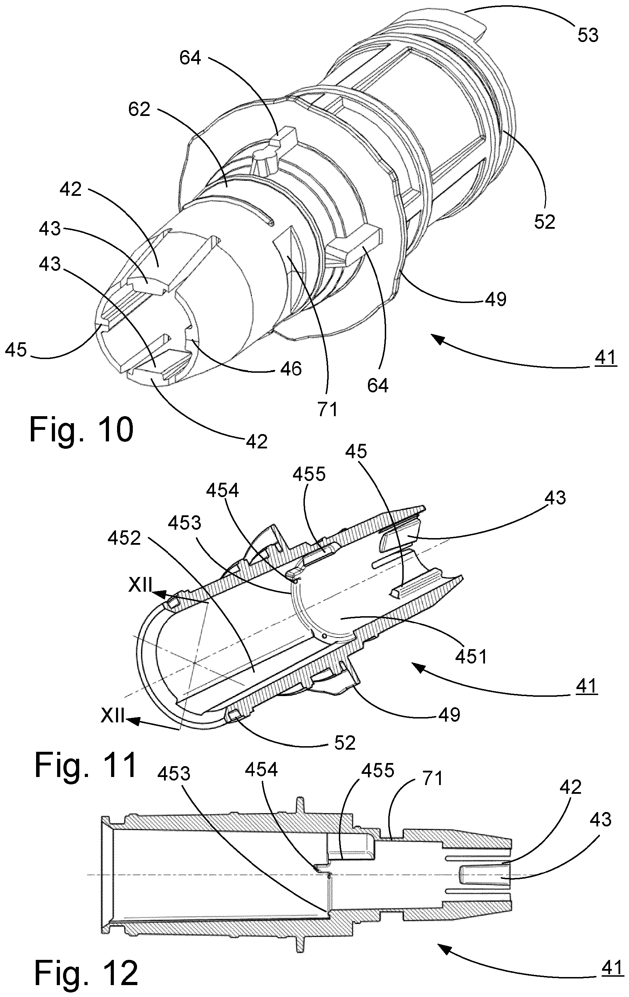

The connection member 28 may then be inserted into a bushing 41. The bushing is made of a low friction material such as POM. This is done by sliding the busing 41 over the insertion cord sub-assembly from the distal end and onto the connection member 28 where it clicks into position, as seen in Figs. 4 and 5. POM is difficult to get adhesive to stick to and accordingly the connection member 28 and the bushing 41 comprise mutually interlocking features securing them with respect to each other in both the circumferential direction as well as in the longitudinal direction. Thus, as can be seen from Fig. 10 the distal end of the bushing 41 comprises two resilient arms 42 with inwardly extending protrusions 43 adapted to elastically snap into engagement with recesses 44 in the outer surface of the connection member 28, thus mutually securing the connection member 28 and the bushing 41 with respect to another. To ensure the correct orientation in the circumferential direction, i.e. ensuring that electrical wiring 16, supply cables and pull wires 19 are later at the correct side within the handle ribs, the inner bushing wall surface 451 is provides with a first rib 45 and a second rib 46 of differing widths, where the first rib 45 is adapted to engage in the slit 36 in the connection member 28 and the second rib 46 is adapted to engage into a groove preferably arranged diametrically opposite side of slit in the outer surface of the connection member 28 (not visible in the figures). This also further secures the connection member 28 and the bushing with respect to another in the circumferential direction.

To end correctly up in this correct orientation in the circumferential direction, the proximal part of the inner bushing wall surface 451 comprises a longitudinal groove 452 adapted to guide a protrusion 281 on a flange 282 on the connection member 28, cf. Fig. 7. When sliding the bushing 41 over the connection member the flange 282 abuts a stop wall 453 formed by a step covering a majority of the circumference of the inner wall surface 451. The position of this stop wall 453 in the longitudinal direction is so selected that the inward extending protrusions 43 latch into the recesses 44 and secure the mutual position of the connection member 28 and the bushing 41 in the longitudinal direction. At the end of the stop wall 453 small protrusions 454 in the longitudinal direction are provided, so as to secure further the position of the connection member 28 with respect to the bushing 41 in the circumferential direction. For the remainder of the circumference, i.e. the minority part between the protrusions 454 the step is missing, so as to provide lateral space 455 for the wires protruding from the opening 37 in the connection member 28 in the assembled state.

The printed circuit board 21 with electrical wiring 16, supply cables and pull wires 19 emerging from the opening 37 into the lateral space 455 and out of the proximal end of the bushing 41 may then be placed in housing part 20 using the slit 23 to pass the cables. A marker ring 47 may be slid over the end of the housing part 20, snap into a recess 48 and partially close the slit 23. More specifically the marker ring 47 may be slid from the distal end of the insertion cord sub-assembly over the bushing 41 to surround the coupling mechanism and preventing expansion of the slit 23 by forces from the busing 41, thus later aiding in ensuring the desired amount of friction between the handle housing part 20 and the bushing 41.

The bushing 41, now connected to the insertion cord sub-assembly may then be inserted into the distal end of the handle housing now partially closed with the marker ring 47. In this position the bushing is secured against axial movement by a flange 49 provided on the outer bushing wall surface 62, and engaging the distal end surface 50 of the housing part 20, and a C-shaped clip 51 engaging in a circumferential recess 52 in the outer surface of the bushing 41. Protruding from the proximal end of the bushing 41 a stop member 53 is preferably provided. This stop member 53 is adapted to engage suitable uprights 54 on the interior surface of the housing part 20 to limit the rotation of the bushing 41 with respect to the handle 5. The uprights 54 may preferably also serve as anchoring points for the Y-junction body 25, i.e. points for adhering the Y-junction body 25 to the interior of the housing part 20.

As mentioned, the bushing 41 is preferably made from a low friction material. To provide for good grip for the operator needing to turn the bushing 41 and the insertion cord 6 with respect to the handle 5, an annular gripping member 14 is provided. The annular gripping member 14 has an external circumferential gripping surface 56 and an inner gripping member surface 57. Preferably the external circumferential gripping surface 56 comprises a number of teeth 59, 60 of which one tooth 60 preferably is higher than the others and/or provided with additional surface features 61 to provide the user with a sense of position. Like the other parts, the annular gripping member 14 is slid over the insertion cord sub-assembly from the distal end thereof.

The inner surface is preferably provided with longitudinal grooves 58 adapted to match external ribs 64 on the outer surface wall 62 of the bushing 41, so as to ensure their correct mutual orientation when assembling the endoscope 1, e.g. so as to have the tooth 60 extending correctly when in a neutral position with respect to the handle 5. Thus, at least one first inner gripping member surface part in the form of the longitudinal grooves 58 is providing engagement with said outer bushing wall 62.

Furthermore, a second inner gripping member surface wall part 63 is rotatably engaging the exterior of said housing part 20. The second inner gripping member surface part 63 comprises at least one, but preferably two, position reference members, e.g. in the form of a notch 63a adapted to mutually engage complementary position reference members 65 on the exterior of the housing part 20. Alternatively, the position reference member 65 on the exterior of the housing part 20 could be provided as a notch. The two position reference members are preferably located with a 180° spacing, in particular with 90° spacing from the tooth 60 on either side thereof. Similarly, the complementary position reference members 65 are located with a 180° spacing on either side of the housing part 20, in particular with 90° spacing from the slit 23 on either side thereof. The complementary position reference members 65 are preferably provided on leaf spring members 66 provided at the distal end of the housing part 20. The leaf spring members 66 may conveniently be formed integrally with the housing part 20 by provision of suitable circumferential slits 67 in the wall of the housing part 20. When aligned the reference members with their complementary parts, under the spring force from the leaf spring members, keeping insertion cord 6b in position with respect to the handle 5 until a user's attempt to turn the insertion cord 6b provides a force overcoming the forces of the leaf spring members 66.

To axially secure the annular gripping member 14 with respect to the bushing 41, the distal end of the annual gripping member 14 comprises a pair of elastic latching members 69 with inward protrusions 70 adapted to engage a corresponding pair of recesses 71 in the outer surface of the bushing 41.

Finally, to complete the assembly of the insertion cord sub-assembly to the handle 5 via the rotary coupling mechanism, a tension relief member 67 may be slid over the insertion cord 6 from the distal end thereof an onto the annular gripping member 14 to engage a generally circumferential ribs 72 at the elastic retaining members 69 on the distal end of the annular gripping member 14. The tension relief member 67 not only serves as tension relief but *inter alia* serves as sealing cover for the coupling mechanism and as a holding means for e.g. an endotracheal tube during intubation of a patient or for a protective tubular cover during storage of the endoscope 1, which may be held by a number of circumferential elastic ribs 68.

In this way a rotary coupling mechanism for an endoscope 1 has been provided in a manner easy to manufacture and easy to assemble.

## Claims

1. An endoscope (1) comprising a proximal handle (5) comprising a housing part (2) and a distal insertion cord (6) coupled to said proximal handle (5) by means of a rotary coupling mechanism allowing rotation about a rotation axis, said coupling mechanism comprising
a bushing (41) having a through aperture defining an inner bushing wall surface (451) adapted to receive a connection member (28) and an outer bushing wall surface (62) adapted to engage an inner handle surface in said handle (5),
a connection member (28) received in said through aperture and having a wall (29) defining a through passage between a first end (34) and a second end (35),
wherein said second end (35) is attached to an insertion cord sub-assembly, and said first end (34) is rotatably coupled to a working channel part (25) within said handle (5),
**characterized in that** a lateral exit opening (37) is provided in said wall (29) between said first end (34) and said second end (35) and
wherein a slit (36) is provided in said wall (29) from said second end (35) to said exit opening (37).

2. An endoscope (1) according to claim 1, wherein the inner bushing wall surface comprises a rib (45) protruding into said slit (36).

3. An endoscope (1) according to claim 1 or 2, wherein the coupling mechanism further comprises an annular gripping member (14) having an external circumferential gripping surface (56) and an inner gripping member surface (57), where the inner gripping member surface (57) comprises a first inner gripping member surface part (58) in engagement with said outer bushing wall surface (62) and a second inner gripping member surface part (63) rotatably engaging the exterior of said housing part (20).

4. An endoscope (1) according to any one of the preceding claims, further comprising a locking clip (51) securing said bushing (41) against axial motion with respect to the housing (20) part along said rotation axis.

5. An endoscope (1) according to claim 4, wherein the locking clip is made from the same material as the bushing (41).

6. An endoscope (1) according to any one of claims 3 to 5 where in the second inner gripping member surface part (63) comprises at least one position reference member (63a) adapted to mutually engage a complementary position reference member (65) on the exterior of said housing part (20).

7. An endoscope (1) according to claim 6, wherein the complementary position reference member (65) is provided on a spring member (66) provided integrally with the housing part (20), said spring member (66) extending in a circumferential direction at the distal end of the housing part (20).

8. A system comprising a display device (2) and an endoscope (1) according to any one of the preceding claims connectable to said display device (2).

## Patentansprüche

1. Endoskop (1), umfassend einen proximalen Griff (5), der ein Gehäuseteil (2) umfasst, und ein distales Einführkabel (6), das mithilfe eines Drehkopplungsmechanismus mit dem proximalen Griff (5) gekoppelt ist, was die Drehung um eine Drehachse erlaubt, wobei der Kopplungsmechanismus umfasst
eine Buchse (41) mit einer Durchgangsöffnung, die eine Buchseninnenwandfläche (451) definiert, die dazu angepasst ist, ein Verbindungselement (28) aufzunehmen, und eine Buchsenaußenwandfläche (62), die dazu angepasst ist, eine Griffinnenfläche in dem Griff (5) in Eingriff zu nehmen,
ein Verbindungselement (28), das in der Durchgangsöffnung aufgenommen ist und eine Wand (29) hat, die einen Durchgang zwischen einem ersten Ende (34) und einem zweiten Ende (35) definiert,
wobei das zweite Ende (35) an einer Einführkabelunteranordnung angebracht ist, und wobei das erste Ende (34) drehbar mit einem Arbeitskanalteil (25) in dem Griff (5) gekoppelt ist,
**dadurch gekennzeichnet, dass** eine seitliche Austrittsöffnung (37) in der Wand (29) zwischen dem ersten Ende (34) und dem zweiten Ende (35) bereitgestellt ist und
wobei ein Schlitz (36) in der Wand (29) von dem zweiten Ende (35) bis zu der Austrittsöffnung (37) bereitgestellt ist.

2. Endoskop (1) nach Anspruch 1, wobei die Buchseninnenwandfläche eine Rippe (45) umfasst, die in den Schlitz (36) vorspringt.

3. Endoskop (1) nach Anspruch 1 oder 2, wobei der Kopplungsmechanismus ferner ein ringförmiges Greifelement (14) mit einer äußeren Umfangsgreiffläche (56) und einer inneren Greifelementfläche (57) umfasst, wobei die innere Greifelementfläche (57) einen ersten inneren Greifelementflächenteil (58) im Eingriff mit der Buchsenaußenwandfläche (62) und einen zweiten inneren Greifelementflächenteil (63) umfasst, der drehbar die Außenseite des Gehäuseteils (20) in Eingriff nimmt.

4. Endoskop (1) nach einem der vorhergehenden Ansprüche, ferner umfassend eine Verriegelungsklammer (51), die die Buchse (41) gegen die axiale Bewegung mit Bezug auf den Gehäuseteil (20) entlang der Drehachse sichert.

5. Endoskop (1) nach Anspruch 4, wobei die Verriegelungsklammer aus demselben Material hergestellt ist wie die Buchse (41).

6. Endoskop (1) nach einem der Ansprüche 3 bis 5, wobei das zweite Greifelementinnenflächenteil (63) mindestens ein Positionsbezugselement (63a) umfasst, das dazu angepasst ist, mit einem komplementären Positionsbezugselement (65) an der Außenseite des Gehäuseteils (20) ineinanderzugreifen.

7. Endoskop (1) nach Anspruch 6, wobei das komplementäre Positionsbezugselement (65) an einem Federelement (66) bereitgestellt ist, das einstückig mit dem Gehäuseteil (20) bereitgestellt ist, wobei sich das Federelement (66) in eine Umfangsrichtung an dem distalen Ende des Gehäuseteils (20) erstreckt.

8. System, umfassend eine Anzeigeeinheit (2) und ein Endoskop (1) nach einem der vorhergehenden Ansprüche, das mit der Anzeigeeinheit (2) verbindbar ist.

## Revendications

1. Endoscope (1) comprenant une poignée proximale (5) comprenant une partie boîtier (2) et un cordon d'insertion distal (6) couplé à ladite poignée proximale (5) au moyen d'un mécanisme de couplage rotatif permettant la rotation autour d'un axe de rotation, ledit mécanisme de couplage comprenant
une bague (41) ayant une ouverture traversante définissant une surface de paroi de bague interne (451) adaptée pour recevoir un élément de raccordement (28) et une surface de paroi de bague externe (62) adaptée pour venir en prise avec une surface de poignée interne dans ladite poignée (5),
un élément de raccordement (28) reçu dans ladite ouverture de passage traversant et ayant une paroi (29) définissant un passage entre une première extrémité (34) et une seconde extrémité (35),
ladite seconde extrémité (35) étant fixée à un sous-ensemble de cordon d'insertion, et ladite première extrémité (34) étant couplée de manière rotative à une partie de canal de travail (25) à l'intérieur de ladite poignée (5),
**caractérisé en ce qu'**une ouverture de sortie latérale (37) est prévue dans ladite paroi (29) entre ladite première extrémité (34) et ladite seconde extrémité (35) et
une fente (36) étant prévue dans ladite paroi (29) de ladite seconde extrémité (35) à ladite ouverture de sortie (37).

2. Endoscope (1) selon la revendication 1, la surface de paroi de bague interne comprenant une nervure (45) faisant saillie dans ladite fente (36).

3. Endoscope (1) selon la revendication 1 ou 2, le mécanisme de couplage comprenant en outre un élément de préhension annulaire (14) ayant une surface de préhension circonférentielle externe (56) et une surface d'élément de préhension interne (57), la surface d'élément de préhension interne (57) comprenant une première partie de surface d'élément de préhension interne (58) en prise avec ladite surface de paroi de bague externe (62) et une seconde partie de surface d'élément de préhension interne (63) en prise rotative avec l'extérieur de ladite partie boîtier (20).

4. Endoscope (1) selon l'une quelconque des revendications précédentes, comprenant en outre un clip de verrouillage (51) fixant ladite bague (41) contre un mouvement axial par rapport à la partie boîtier (20) le long dudit axe de rotation.

5. Endoscope (1) selon la revendication 4, le clip de verrouillage étant constitué du même matériau que la bague (41).

6. Endoscope (1) selon l'une quelconque des revendications 3 à 5, la seconde partie de surface d'élément de préhension interne (63) comprenant au moins un élément de référence de position (63a) adapté pour venir en prise mutuellement avec un élément de référence de position complémentaire (65) sur l'extérieur de ladite partie boîtier (20).

7. Endoscope (1) selon la revendication 6, l'élément de référence de position complémentaire (65) étant prévu sur un élément de ressort (66) fourni de manière intégrée avec la partie boîtier (20), ledit élément de ressort (66) s'étendant dans une direction circonférentielle à l'extrémité distale de la partie boîtier (20).

8. Système comprenant un dispositif d'affichage (2) et un endoscope (1) selon l'une quelconque des revendications précédentes, pouvant être connecté audit dispositif d'affichage (2).
